# EUROPEAN PATENT APPLICATION

(11) **EP 4 800 569 A1**
(43) Date of publication of application: **02.09.2026**
(21) Application number: 26155820.9
(22) Date of filing: 02.02.2026
(51) Int. Cl.: G06F 16/22, G06F 16/25, G06F 16/334, G16H 10/60, G16H 50/70, G06F 16/2455

(54) **FEDERATED VECTOR DATABASE SYSTEM**

(30) Priority: 26.02.2025 US 202519064412
(71) Applicant: VANTIQ, INC., Walnut Creek, CA 94596 (US)
(72) Inventor: BUTTERWORTH, Paul, Alamo, 94507 (US)
(74) Representative: Murgitroyd & Company

(57) **Abstract**

A system and method including receiving, via a query interface, a query related to a medical topic, transmitting the query to one or more vector DBs, and retrieving result sets from the one or more vector DBs, each result set associated with a vector DB of the one or more vector DBs, each result set including result records, each result record being associated with content and a respective content vector. The system normalizes, based on a vectorization algorithm, one or more of the result records in the result sets to generate a normalized result set. The system generates, based on the normalized result set and one or more parameters, a unified result set, stores the unified result set, and returns the unified result set to a medical decision system that provides medical recommendations to a system user.

## Description

### CLAIM OF PRIORITY

This application claims the benefit of priority to U.S. Application Serial No. 19/064,412, filed on February 26, 2025.

### TECHNICAL FIELD

The disclosed subject matter relates generally to the technical field of query-answering systems and, in one specific example, to a solution for answering queries by querying multiple vector databases.

### BACKGROUND

Given the growing number of data sources with different schemas and/or different access protocols, significant effort has gone into developing automatic query-answering systems that can efficiently answer queries based on leveraging data from across such data sources.

### SUMMARY

The invention is an apparatus, a method and a computer-readable storage medium as defined in the appended claims.

### BRIEF DESCRIPTION OF SEVERAL VIEWS OF THE DRAWINGS

To easily identify the discussion of any particular element or act, the most significant digit or digits in a reference number refer to the figure number in which that element is first introduced.
FIG. 1 is a network diagram depicting a system within which various examples described herein may be deployed.
FIG. 2 is a diagrammatic representation of a federated vector database (DB) system, according to some examples.
FIG. 3 is a flowchart that illustrates a method implemented by a federated vector DB system, according to some examples.
FIG. 4 is a block diagram showing a machine-learning (ML) program, according to some examples.
FIG. 5 is a block diagram illustrating an example of a software architecture that may be installed on a machine, according to some examples.
FIG. 6 is a block diagram illustrating components of a machine, according to some examples, able to read instructions from a machine-readable medium and perform any one or more of the methodologies discussed herein.

### DETAILED DESCRIPTION

Developing automatic query-answering systems is an area of significant investment, with efforts leveraging recent breakthroughs in Large Language Models (LLMs) as well as vector databases (vector DBs). Vector DBs can be used to augment statically trained LLMs with recent data, or with proprietary data not available for model training. In some examples, vector DBs can exist throughout a distributed computing environment. Individual vector DBs can be maintained on edge nodes containing, for example, information derived from devices directly connected to the edge node. In some examples, vector DBs can be replicated on multiple edge nodes to provide fault tolerance and/or local autonomy in various systems. Individual vector DBs can also be maintained in regional compute centers. For example, a hospital network maintains one or more vector DBs at each hospital representing data that is collected at the local hospital or data that is used locally without depending on network connectivity.

A vector DB uses a vectorization algorithm, such as an encoding or embedding method, to generate vectors associated with content items (e.g., documents, structured data entries, images, videos, and so forth). The vector DB can store records associated with the content and/or the content vector (e.g., content item embeddings). Given a query, the vector DB uses the same vectorization algorithm to compute a query vector (e.g., query embedding). The vector DB then executes a search of stored content vectors. The vector DB can compute a distance between each stored content vector and the query vector. The vector DB can return the closest K content vectors (K = constant, K >=1). Thus, given a query, the vector DBs retrieve semantically similar and or relevant stored data or content if the content vectors accurately represent the semantics of the original content.

Automatic query answering systems frequently access not just one vector DB, but a set of multiple vector DBs that potentially use different vectorization algorithms and/or have different access protocols. Thus, result records returned by different vector DBs may not be able to be directly compared to each other. Furthermore, distances between retrieved content vectors and the query vector may not be directly comparable across DBs. Therefore, given a query answering system with access to multiple vector DBs, there is a need for an answer generation solution that allows for comparing query result records returned by different vector DBs even in the presence of different vectorization algorithms. Such a solution would enable an automatic query answering system to leverage data from a heterogeneous vector DB set, and/or provide comprehensive, accurate result sets to a querying agent and/or API.

Examples described herein refer to a federated vector DB system that enables a querying agent and/or API to transmit a query and/or receive one or more sets of result records retrieved from one or more vector DBs that use potentially different vectorization algorithms. In some examples, the result records include results ranked according to a relevance metric with respect to the input query. In some examples, the relevance metric corresponds to a distance in a vector space between a query vector associated with the query and a content vector associated with a content item of a specific result record. In some examples, the federated vector DB system addresses the issue of differing vectorization algorithms for the different participant vector DBs by using a single, chosen vectorization algorithm to re-vectorize the elements of the one or more retrieved result sets across the participant vector DBs. The same vectorization algorithm is used to compute a vector of the input query. Given the re-vectorized result set members and the input query, the federated vector DB system can rank the result records in the result sets. For example, the federated vector DB system can use computed distances in the new vector space between the query vector and each of the new content vectors for the result records. The federated vector DB system can then output a set of most relevant results to the querying agent and/or API.

In some examples, the federated vector DB system receives a query via a query interface. The federated vector DB system transmits the query to one or more vector DBs, and retrieves one or more result sets from the one or more vector DBs. Each result set associated with a vector DB includes result records and each result record is associated with content and a respective content vector. The federated vector DB system normalizes, based on a vectorization algorithm, one or more of the result records in the result sets to generate a normalized result set. Normalizing the one or more of the result records includes generating, using the vectorization algorithm, a new content vector for the content associated with each of the one or more of the result records.

The federated vector DB system generates, based on the normalized result set and one or more parameters, a unified result set, stores the unified result set, and/or returns the unified result set to a querying agent and/or API. Generating a unified result set can include ranking result records in the normalized result set based on relevance to the query in order to generate a ranked result set. The unified result set can be generated based on the ranked result set and the one or more parameters of the query interface (e.g., a maximum result set size, a minimum set relevance score, and so forth).

In some examples, the federated vector DB system can implement one or more optimizations. For example, when detecting that the vectorization algorithm matches a local vectorization algorithm of a vector DB, result records of a result set retrieved from the respective vector DB can be directly added to the normalized result set. Furthermore, the federated vector DB system can use a cache, for example in order to avoid re-vectorizing already re-processed local results. The cache can also maintain timestamps, ensuring that cache entries are aged out over time and/or keeping the size of the cache from continuously increasing.

In some examples, the input query is a query related to a medical topic, and the unified result set is returned to a medical decision system that provides medical recommendations to a system user. For example, the federated vector DB system could allow medical personnel (e.g., doctors, administrative personnel, etc.) access to patient data collected from multiple sources, such as wearable health monitors, hospital databases, and/or regional health data repositories. The federated vector DB system could process queries related to patient health indicators and retrieve comprehensive and relevant data useful for making medical decisions. Such data can further be provided to a medical decision making system and/or component.

In some examples, the federated vector DB system can be used in industrial settings. For example, Internet of Things (IOT) sensors for machinery can collect data related to temperature, vibration, acoustics, and so forth, where the data is stored in a collection of individual vector DBs. Queries can involve predicting machinery failures, scheduling maintenance, and so forth.

In some examples, the federated vector DB system can be used in a smart home environment. IoT devices (e.g., lighting systems, smart thermostats, security cameras, appliances, etc.) collect data corresponding to user interactions, environmental conditions, device status, and so forth. Local vector DBs can be used to store such data for further access. Queries to the federated vector DB system can refer to device energy use, temperature settings based on occupancy and/or time of day, and so forth. The results can be provided to a system or downstream component that recommends, to a user or agent, ways to optimize energy usage, improve user comfort by automatically adjusting device settings, and so forth.

In some examples, the federated vector DB system can be used in the financial sector, for example to enhance fraud detection and/or risk management. By integrating data from various financial institutions and transaction databases, the system can analyze patterns and detect anomalies that may indicate fraudulent activities, process queries related to transaction behaviors, account movements, and other financial indicators to provide a comprehensive risk assessment, helping financial institutions mitigate potential losses.

In some examples, the federated vector DB system can be used to optimize logistics and inventory management across different locations and/or databases. By querying multiple vector DBs that store information on inventory levels, shipment status, and/or supplier performance, the system can help with item reordering, shipment route planning, and/or identifying reliable suppliers.

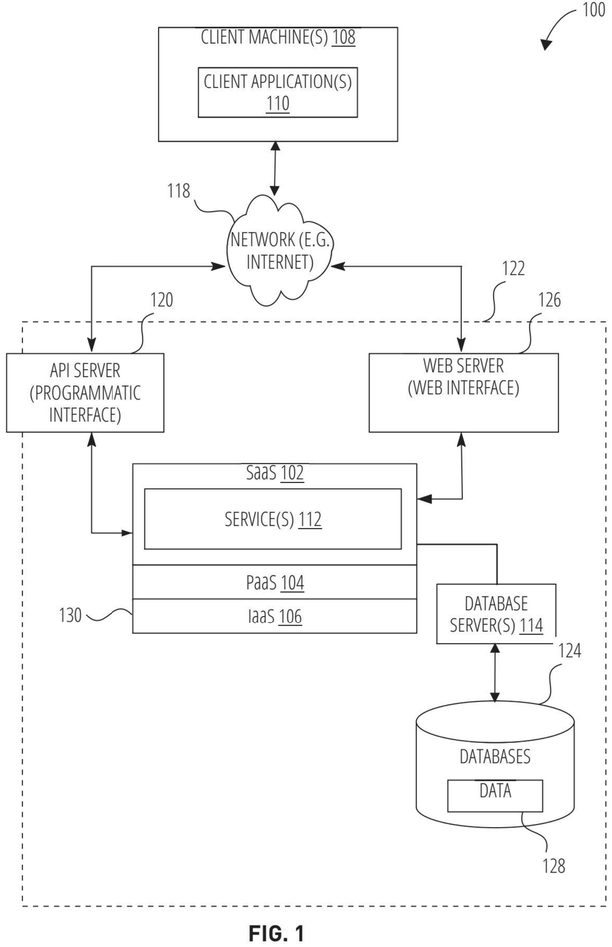

FIG. 1 is a network diagram depicting a system 100 within which various examples described herein may be deployed. A networked system 122, in the example form of a cloud computing service, such as Microsoft Azure or other cloud service, provides server-side functionality, via a network 118 (e.g., the Internet or Wide Area Network [WAN]) to one or more endpoints (e.g., client machine(s) 108). FIG. 1 illustrates client application(s) 110 on the client machine(s) 108. Examples of client application(s) 110 may include a web browser application, such as the Internet Explorer browser developed by Microsoft Corporation of Redmond, Washington or other applications supported by an operating system of the device, such as applications supported by Windows, iOS or Android operating systems. Examples of such applications include e-mail client applications executing natively on the device, such as an Apple Mail client application executing on an iOS device, a Microsoft Outlook client application executing on a Microsoft Windows device, or a Gmail client application executing on an Android device. Examples of other such applications may include calendar applications, file sharing applications, contact center applications, digital content creation applications (e.g., game development applications) or game applications. Each of the client application(s) 110 may include a software application module (e.g., a plug-in, add-in, or macro) that adds a specific service or feature to the application.

An API server 120 and a web server 126 are coupled to, and provide programmatic and web interfaces respectively to, one or more software services, which may be hosted on a software-as-a-service (SaaS) layer or platform 102. The SaaS platform may be part of a service-oriented architecture, being stacked upon a platform-as-a-service (PaaS) layer 104 which, in turn, may be stacked upon an infrastructure-as-a-service (IaaS) layer 106 (e.g., in accordance with standards defined by the National Institute of Standards and Technology [NIST]).

While the applications (e.g., service(s)) 112 are shown in FIG. 1 to form part of the networked system 122, in alternative embodiments, the applications 112 may form part of a service that is separate and distinct from the networked system 122.

Further, while the system 100 shown in FIG. 1 employs a cloud-based architecture, various embodiments are, of course, not limited to such an architecture, and could equally well find application in a client-server, distributed, or peer-to-peer system, for example. The various server services or applications 112 could also be implemented as standalone software programs. Additionally, although FIG. 1 depicts machine(s) 108 as being coupled to a single networked system 122, it will be readily apparent to one skilled in the art that client machine(s) 108, as well as client application(s) 110 (such as game applications), may be coupled to multiple networked systems, such as payment applications associated with multiple payment processors or acquiring banks (e.g., PayPal, Visa, MasterCard, and American Express).

Web applications executing on the client machine(s) 108 may access the various applications 112 via the web interface supported by the web server 126. Similarly, native applications executing on the client machine(s) 108 may access the various services and functions provided by the applications 112 via the programmatic interface provided by the API server 120. For example, the third-party applications may, utilizing information retrieved from the networked system 122, support one or more features or functions on a website hosted by the third party. The third-party website may, for example, provide one or more promotional, marketplace or payment functions that are integrated into or supported by relevant applications of the networked system 122.

The server applications may be hosted on dedicated or shared server machines (not shown) that are communicatively coupled to enable communications between server machines. The server applications 112 themselves are communicatively coupled (e.g., via appropriate interfaces) to each other and to various data sources, so as to allow information to be passed between the server applications 112 and so as to allow the server applications 112 to share and access common data. The server applications 112 may furthermore access one or more databases 124 via the database server(s) 114. In some examples, various data items are stored in the databases 124, such as the system's data items 128. In some examples, the system's data items may be any of the data items described herein.

Navigation of the networked system 122 may be facilitated by one or more navigation applications. For example, a search application (as an example of a navigation application) may enable keyword searches of data items included in the one or more databases 124 associated with the networked system 122. A client application may allow users to access the system's data 128 (e.g., via one or more client applications). Various other navigation applications may be provided to supplement the search and browsing applications.

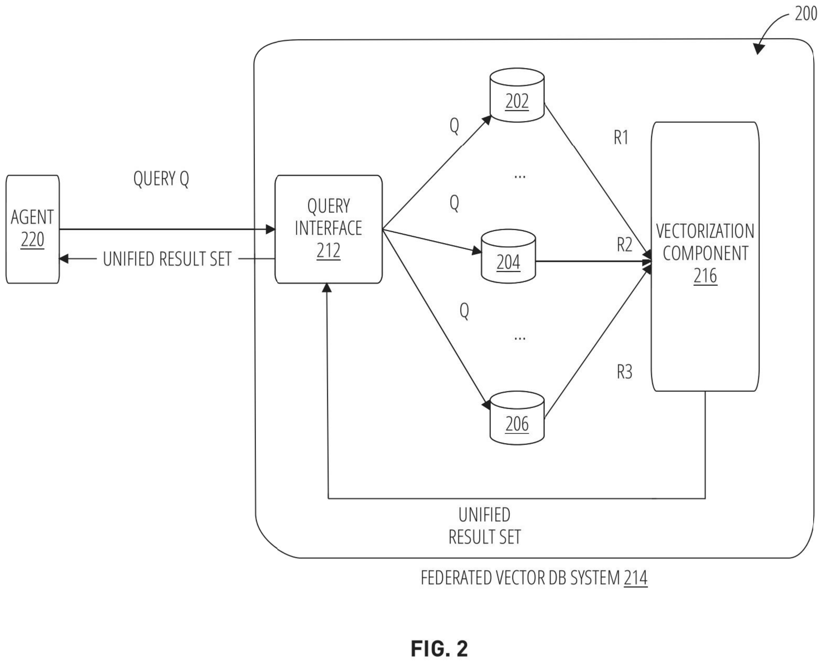

FIG. 2 is a diagrammatic representation 200 of a federated vector DB system 214 according to some examples. The federated vector DB system 214 includes a query interface 212 component, a set of individual databases (e.g., 202, 204, 206), a vectorization component 216, and/or other components as described below, such as for example a cache component. In some examples, the federated vector DB system 214 corresponds to a single virtual database with accompanying functionality from the point of view of a requesting and/or querying agent (e.g., a system, an API, a developer, etc.), as seen below.

The federated vector DB system 214 encapsulates or includes one or more individual vector DBs (e.g., 202, 204, 206, etc.) The one or more individual vector DBs can be defined, in the configuration of the federated vector DB system, as a single virtual vector DB. Given a query q from agent 220, the federated vector DB system 214 receives the query at query interface 212 and/or processes them to be further used by the federated vector DB system 214. Given the query q, the federated vector DB system 214 returns to the querying agent a result set including the records or results that best satisfy the query, the records being retrieved from the one or more vector DBs included in the federated vector DB system.

### Individual Vector DBs

In some examples, an individual vector DB (e.g., 202, 204, 206) accesses a record containing content (e.g., one or more documents, images, videos, audio, multi-modal content items, etc.). The vector DB computes a vector associated with the specific content, thereby mapping the content to a point or position in an N-dimensional vector space. The record can be augmented to include the content vector.

Given a query, the individual vector DB converts the query's content into a query vector (e.g., a query embedding). The vector DB executes a search of stored content vectors. The vector DB computes a distance, in the N-dimensional space, between each stored content vector and the query vector. The vector DB returns the closest K content vectors (where K is a constant, K >=1). Given a query, vector DBs retrieve semantically similar and or relevant stored data or content if the content vectors accurately represent the semantics of the original content.

In some examples, a vector DB maintains an authentication system for authenticating requests. The authentication system can be used to update the content of the vector DB, or to query the vector DB. A vector DB can use one of a number of embedding and/or encoding algorithms (e.g., vectorization algorithms) to map content to a vector. Example embedding algorithms and/or frameworks include: Word2Vec, Doc2Vec, FastText, CLIP, SimCLR, VGGish, ViLBERT, and more. A vector DB can be accessed and/or queried via a dedicated query interface with a given UI and/or API.

Thus, individual vector DBs used by a federated vector DB system 214 can have multiple, distinct authentication systems, vectorization algorithms and/or query interface configurations and/or APIs.

### A Federated Vector DB System

As mentioned above, the federated vector DB system 214 can assemble and/or encapsulate federated vector DBs. Given an individual vector DB, the federated vector DB system 214 maintains all or a subset of the following information:
- The identity (ID) of the vector DB.
- Information about the vectorization algorithm used by the vector DB.
- A schema describing the structure of metadata if the vector database stores metadata associated with the content in addition to the content itself.
- An access point or endpoint associated with the vector DB (e.g., used to receive or access a query, and/or retrieve a result), and/or an address associated with the access point.
- A description of a query interface used to query the vector DB.
- A set of credentials to be used to access the vector DB.

In some examples, the federated vector DB system 214 advertises an access point, associated for example with a query interface 212. The federated vector DB system 214 can process queries in a format pre-defined by the federated vector DB system 214, and/or can require that a query and/or querying agent present the system with credentials that authorize access to the federated vector DB system 214.

### Answering Queries in a Federated Vector DB System

In some examples, the query interface 212 receives a query q from agent 220, where the query follows the query format described in a configuration of the federated vector DB system 214 (as described above). In some examples, the federated vector DB system 214 verifies that the agent 220 has credentials enabling it to receive access to the federated vector DB system 214.

Given query q, the federated vector DB system 214 determines a set of vector databases in a query set (e.g., 202, 204, etc.). For each vector database in the query set, the federated vector DB system 214 determines a corresponding endpoint address (e.g., the address of an access point) and/or access credentials. The federated vector DB system 214 submits query q to each vector database in the query set, and retrieves a result (e.g., result set R1, result set R2, etc.). The federated vector DB system 214 automatically combines one or more of the retrieved result sets into a unified result set, which is returned, for example via the query interface 212, to the requesting agent 220. Thus, the requesting agent 220 interacts with the federated vector DB system 214 similarly, from its perspective, to interacting with a single vector database.

In some examples, the unified result set includes records retrieved from the one or more independent vector DBs such that the K records are associated with content and/or content vectors that are most relevant to the query and/or query vector. In some examples, relevance is computed based on how closely a retrieved content vector associated with content for a particular record is to a query vector associated with the query.

In some examples, the query interface 212 uses one or more parameters to control the size of the unified result set, the one or more parameters including at least:

### a) maximum result set size

In some examples, the number of local or individual vector DBs can exceed M (M = constant, M > 1).

If agent 220 requests and/or receives results from a federated vector DB system 214 using a large enough M, the unified results set can be too large for the querying agent 220 to process efficiently. Thus, agent 220 can specify a maximum result size (e.g., N, with N > 0) via a dedicated parameter of the query interface 212. The federated vector DB system 214 limits the result set to the N most relevant records found across the federated set of vector DBs.

### b) minimum result set relevance

In some examples, agent 220 can set, via a parameter of the query interface 212, a minimum result set relevance (e.g., a threshold T, where T is a constant, T > 0). In some examples, the minimum result set relevance threshold corresponds to a maximum distance between a record's content vector and the query vector for the input query q. For example, the distance between the input query vector and the content vector for each entry in the unified result set is determined to be smaller than the minimum result set relevance.

In some examples, the federated vector DB system 214 can use a distributed query algorithm to issue the input query to the individual vector DBs, and/or retrieve and unify the results. However, as indicated above, individual vector DBs can have differing schemas and/or use one or more vectorization algorithms. Thus, results retrieved from the different DBs can correspond to content vectors generated using different vectorization algorithms. Therefore, the content vectors and/or computed content vector-query vector distances cannot be directly compared in order to identify a set of K content vectors most relevant to the input query q.

### Normalizing Query Results in a Federated Vector DB System

In some examples, the technical problem of comparing relevance or closeness metrics for retrieved records associated with content vectors produced by differing vectorization algorithms can be solved by a re-vectorization process that uses a single vectorization algorithm to compute new vectors for content associated with retrieved records.

In some examples, the federated vector DB system 214 selects, for example via its vectorization component 216, the single vectorization algorithm. The vectorization component 216 receives results sets (e.g., R1, R2, etc.) from the individual vector DBs (202, 204, 206) and/or applies the selected single vectorization algorithm to the content associated with result records in the result sets, producing a set of new, directly comparable content vectors.

In some examples, the federated vector DB system 214 can rank the set of new content vectors produced by the single vectorization algorithm with respect to one or more relevance metrics. For example, the federated vector DB system 214 can compute a distance (e.g., cosine similarity, etc.) between the query vector for query input q and content vectors in the set of new content vectors. These computed distances are directly comparable, given that the new content vectors are in the same encoding and/or embedding space. Thus, the federated vector DB system 214 can return to the querying agent 220 a set of result records ranked, for example, in order of relevance or closeness with respect to the input query q.

In some examples, given a maximum result set size N received from agent 220, the federated vector DB system 214 can return a unified result set of N most relevant record results.

In some examples, given a minimum result set relevance score received from agent 220, the federated vector DB system 214 can include in the unified result set only result records whose associated new content vectors meet the relevance threshold (e.g., are closer to the input query vector than the minimum result set relevance score).

### Examples: Optimizations for a Federated Vector DB System

In some examples, the federated vector DB system 214 implements optimizations in order to reduce costs and/or improve query answering speed.

In some examples, the federated vector DB system 214 selects, as the single vectorization algorithm, a vectorization algorithm that is most frequently used in the individual vector databases, or that is used in a subset of the individual vector databases of a minimum predetermined size.

If the single vectorization algorithm selected by the federated vector DB system is the same as the vectorization algorithm for one of the individual vector DBs in the federated vector DB system 214, the content in the result records retrieved from the respective individual vector DB need not be re-vectorized (e.g., re-encoded) using the single vectorization algorithm. In some examples, the individual vector DBs include information specifying their used vectorization algorithm in the information maintained by the federated vector DB system 214 about each participating individual vector DB (e.g., profile information specific to each individual vector DB). Therefore, the optimization can be applied based on the available information once the single vectorization algorithm is selected by the federated vector DB.

In some examples, the results of the re-vectorization process include a set of records, each associated with a new content vector corresponding to the content re-encoded using the single vectorization algorithm. These records can be cached, using a cache component. Each cache entry can be indexed by the original content vector (e.g., returned from an individual vector DB). Thus, the cache can include cache indexes, each cash index being associated with a cache entry for a record. Each cash index can correspond to the original content vector for the record (e.g., returned from an individual vector DB, and/or based on content and/or a local vectorization algorithm for the individual vector DB). The cache entry can contain the new content vector (e.g., produced by the vectorization component 216 of the federated vector DB system 214, etc.). In some examples, upon retrieving a record and its associated content vector from a respective individual vector DB, the federated vector DB system 214 can check if the content vector is in the cache (e.g., check if the record is already indexed in the cache based on the original associated content vector). If so, the cached new content vector can be retrieved from the cache and/or used as part of the unified result set.

In some examples, the cache maintains timestamps, ensuring that cache entries are aged out over time and/or keeping the size of the cache from continuously increasing. The federated vector DB system 214 also handles cache entries corresponding to records whose associated content in individual vector DBs has been updated and/or deleted. If a stored record is associated with content that has been updated in the original vector DB, it will not be retrieved via an indexing check, since the updated content will result in an updated content vector. Thus, the cache entry corresponding to the respective record will no longer be retrieved as part of processing the input query. In some examples, records with associated original content that has been deleted in the individual vector DB will no longer be retrieved, as the result set for the individual vector DB will no longer include the respective content vectors for the (now deleted) content.

In some examples, the agent 220 requests results from a federated vector DB system 214 via a query associated with a maximum result set size (e.g., N, with N > 0), as previously discussed. If the specified maximum result set size is bigger than the number of retrieved results for the query, the federated vector DB system 214 can use a merge sort procedure to minimize the number of results or result records to be re-vectorized. For example, the results returned from each individual vector DB can be ranked and/or returned in relevance order, from the highest relevance level to the lowest relevance level. The federated vector DB system 214 can select the result sets corresponding to the individual vector DBs using the same vectorization algorithm as the selected single vectorization algorithm (as seen above) to be used for re-vectorization. The result records in the selected result sets can be ranked based on relevance, from most relevant to least relevant. If the size S of a resulting union of the selected result sets (the union elements ranked by relevance) is equal to or bigger than the requested maximum result set size N, the federated vector DB system 214 can select the top ranked N elements of the resulting union set as the result set for the query. If S < N, the federated vector DB system 214 adds all the result records in the resulting union of selected result sets to the result set for the query.

In some examples, the federated vector DB system 214 selects, for each remaining individual vector DB using a different vectorization algorithm than the selected single vectorization algorithm, a first result record in its associated result set (ranked by relevance of result records) and/or re-vectorizes the result record. If the result set for the query does not yet contain N elements (e.g., corresponding to the maximum result set size), the re-vectorized result is added to the result set. If the result set contains N elements, the federated vector DB system 214 determines if the relevance for the result record is greater than the relevance of at least one item in the result set. If so, the federated vector DB system 214 adds the result record to the result set, and deletes the least relevant item already in the result set. If the relevance of the result record is not greater than that of at least one item in the result set, the federated vector DB system 214 removes the corresponding individual vector DB result set from consideration and/or further processing (e.g., none of the remaining entries in the respective result set will be relevant enough to be included in the result set, as the individual vector DB result set is ranked by relevance). The federated vector DB system 214 proceeds to examine a next most relevant item from an individual vector DB (e.g., the same individual vector DB or a different one), and repeats the above steps. The federated vector DB system 214 thus examines and/or adds to the result set successive results from the individual vector DBs (e.g., DBs using one or more different vectorization algorithms than the selected single vectorization algorithm). The federated vector DB system 214 stops examining and/or adding such results when the size of the result set is the specified maximum result set size.

In some examples, the parameters used by the query interface 212 include a control parameter corresponding to the minimum number of result records that should be included from each individual vector DB. For instance, in some examples the individual vector DBs can represent a subset of an overall population (e.g., each individual DB corresponding to a sub-population). In such cases, a predetermined minimum number of representatives (e.g., result records) from each sub-population may be needed for valid statistical analysis in the context of the query or of a set of queries.

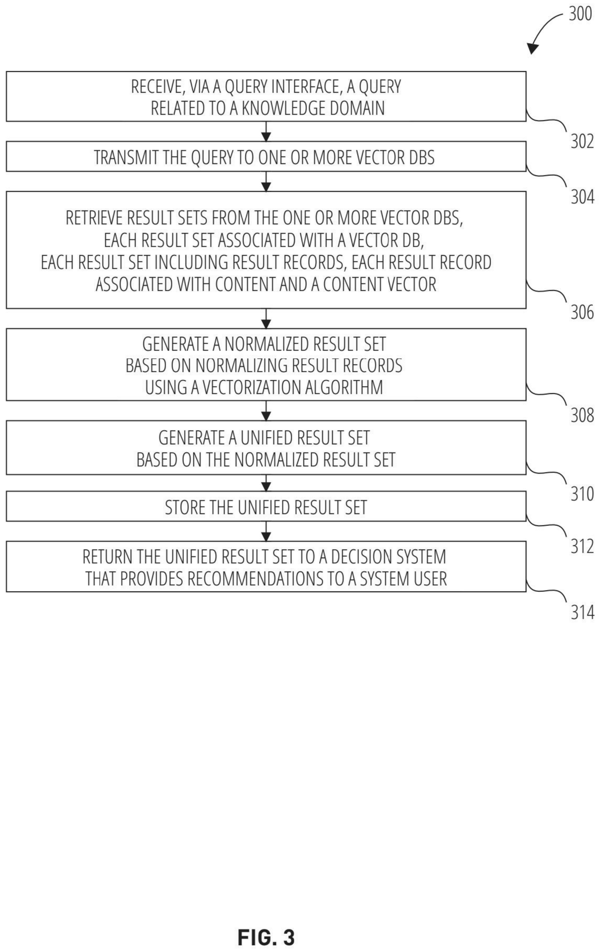

FIG. 3 is a flowchart illustrating a method 300 for query answering in a federated vector DB system, according to some examples. At operation 302, method 300 receives via a query interface, a query related to a knowledge domain (e.g., a medical topic, an industrial or smart home IOT system topic etc.) At operation 304, method 300 transmits the query to one or more vector DBs. At operation 306, method 300 retrieves result sets from the one or more vector DBs, each result set associated with a vector DB of the one or more vector DBs, each result set comprising result records, each result record being associated with content and a respective content vector. At operation 308, method 300 normalizes, based on a vectorization algorithm, one or more of the result records in the result sets to generate a normalized result set. At operation 310, method 300 generates, based on the normalized result set and one or more parameters, a unified result set. At operation 312, method 300 stores the unified result set. In operation 314, method 300 returns the unified result set to a decision system that provides recommendations (e.g., diagnostics, treatment plans, etc.) to a system user.

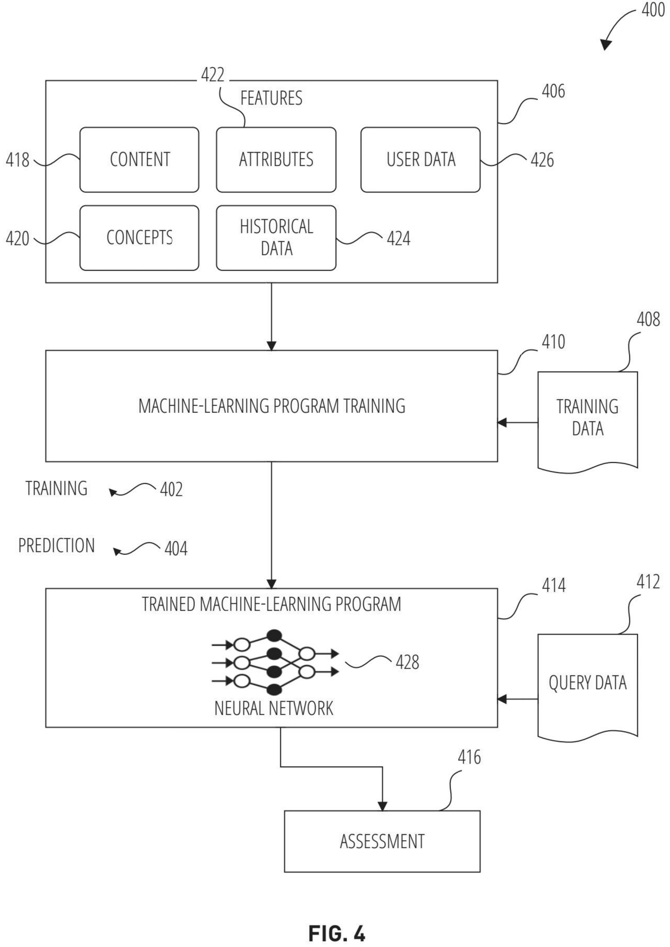

FIG. 4 is a block diagram showing a machine-learning program 400 according to some examples. The machine-learning programs 400, also referred to as machine-learning algorithms or tools, are used to train machine learning models, which can be used by the agents described at least in FIG. 2 of the disclosure herein.

Machine learning (ML) is a field of study that gives computers the ability to learn without being explicitly programmed. Machine learning explores the study and construction of algorithms, also referred to herein as tools, that may learn from or be trained using existing data and make predictions about or based on new data. Such machine-learning tools operate by building a model from example training data 408 in order to make data-driven predictions or decisions expressed as outputs or assessments (e.g., assessment 416). Although examples are presented with respect to a few machine-learning tools, the principles presented herein may be applied to other machine-learning tools.

In some examples, different machine-learning tools may be used. For example, Logistic Regression (LR), Naive-Bayes, Random Forest (RF), Gradient Boosted Decision Trees (GBDT), neural networks (NN), matrix factorization, and Support Vector Machines (SVM) tools may be used. In some examples, one or more ML paradigms may be used: binary or n-ary classification, semi-supervised learning, etc. In some examples, time-to-event (TTE) data will be used during model training. In some examples, a hierarchy or combination of models (e.g. stacking, bagging) may be used.

Two common types of problems in machine learning are classification problems and regression problems. Classification problems, also referred to as categorization problems, aim at classifying items into one of several category values (for example, is this object an apple or an orange?). Regression algorithms aim at quantifying some items (for example, by providing a value that is a real number).

The machine-learning program 400 supports two types of phases, namely a training phase 402 and prediction phase 404. In a training phase 402, supervised learning, unsupervised learning or reinforcement learning may be used. For example, the machine-learning program 400 (1) receives features 406 (e.g., as structured or labeled data in supervised learning) and/or (2) identifies features 406 (e.g., unstructured or unlabeled data for unsupervised learning) in training data 408. In a prediction phase 404, the machine-learning program 400 uses the features 406 for analyzing input or (query) data 412 to generate outcomes or predictions, as examples of an assessment 416.

In the training phase 402, feature engineering is used to identify features 406 and may include identifying informative, discriminating, and independent features for the effective operation of the machine-learning program 400 in pattern recognition, classification, and regression. In some examples, the training data 408 includes labeled data, which is known data for pre-identified features 406 and one or more outcomes. Each of the features 406 may be a variable or attribute, such as individual measurable property of a process, article, system, or phenomenon represented by a data set (e.g., the training data 408). Features 406 may also be of different types, such as numeric features, strings, and graphs, and may include one or more of content 418, concepts 420, attributes 422, historical data 424 and/or user data 426, merely for example.

In training phases 402, the machine-learning program 400 uses the training data 408 to find correlations among the features 406 that affect a predicted outcome or assessment 416.

With the training data 408 and the identified features 406, the machine-learning program 400 is trained during the training phase 402 at machine-learning program training 410. The machine-learning program 400 appraises values of the features 406 as they correlate to the training data 408. The result of the training is the trained machine-learning program 414(e.g., a trained or learned model).

Further, the training phases 402 may involve machine learning (such as deep learning), in which the training data 408 is structured (e.g., labeled during preprocessing operations), and the trained machine-learning program 414 implements a relatively simple neural network 428 (or one of other machine learning models, as described herein) capable of performing, for example, classification and clustering operations. In other examples, the training phase 402 may involve training data 408 which is unstructured, and the trained machine-learning program 414 implements a deep neural network 428 that is able to perform both feature extraction and classification/clustering operations.

A neural network 428 generated or trained during the training phase 402 and implemented within the trained machine-learning program 414, may include a hierarchical (e.g., layered) organization of neurons. For example, neurons (or nodes) may be arranged hierarchically into a number of layers, including an input layer, an output layer, and multiple hidden layers. The layers within the neural network 428 can have one or many neurons, and the neurons operationally compute a small function (e.g., activation function). For example, if an activation function generates a result that transgresses a particular threshold, an output may be communicated from that neuron (e.g., transmitting neuron) to a connected neuron (e.g., receiving neuron) in successive layers. Connections between neurons also have associated weights, which define the influence of the input from a transmitting neuron to a receiving neuron.

In some examples, the neural network 428 may also be one of several different types of neural networks, such as a single-layer feed-forward network, a Multilayer Perceptron (MLP), an Artificial Neural Network (ANN), a Recurrent Neural Network (RNN), a Long Short-Term Memory Network (LSTM), a Bidirectional Neural Network, a symmetrically connected neural network, a Deep Belief Network (DBN), a Convolutional Neural Network (CNN), a Generative Adversarial Network (GAN), an Autoencoder Neural Network (AE), a Restricted Boltzmann Machine (RBM), a Hopfield Network, a Self-Organizing Map (SOM), a Radial Basis Function Network (RBFN), a Spiking Neural Network (SNN), a Liquid State Machine (LSM), an Echo State Network (ESN), a Neural Turing Machine (NTM), or a Transformer Network, merely for example.

During prediction phases 404 the trained machine-learning program 414 is used to perform an assessment. Query data 412 is provided as an input to the trained machine-learning program 400, and the trained machine-learning program 414 generates the assessment 416 as output, responsive to receipt of the query data 412.

In some examples, the trained machine-learning program 414 may be a generative AI model. Generative AI is a term that may refer to any type of artificial intelligence that can create new content from training data 408. For example, generative AI can produce text, images, video, audio, code, or synthetic data similar to the original data but not identical.

Some of the techniques that may be used in generative AI are:
- Convolutional Neural Networks (CNNs): CNNs may be used for image recognition and computer vision tasks. CNNs may, for example, be designed to extract features from images by using filters or kernels that scan the input image and highlight important patterns.
- Recurrent Neural Networks (RNNs): RNNs may be used for processing sequential data, such as speech, text, and time series data, for example. RNNs employ feedback loops that allow them to capture temporal dependencies and remember past inputs.
- Generative adversarial networks (GANs): GNNs may include two neural networks: a generator and a discriminator. The generator network attempts to create realistic content that can "fool" the discriminator network, while the discriminator network attempts to distinguish between real and fake content. The generator and discriminator networks compete with each other and improve over time.
- Variational autoencoders (VAEs): VAEs may encode input data into a latent space (e.g., a compressed representation) and then decode it back into output data. The latent space can be manipulated to generate new variations of the output data. VAEs may use self-attention mechanisms to process input data, allowing them to handle long text sequences and capture complex dependencies.
- Transformer models: Transformer models may use attention mechanisms to learn the relationships between different parts of input data (such as words or pixels) and generate output data based on these relationships. Transformer models can handle sequential data, such as text or speech, as well as non-sequential data, such as images or code.

In generative AI examples, the output prediction/inference data include predictions, translations, summaries or media content.

In some generative AI examples, the trained machine-learning program 414 can be a Large Language Model (LLM). LLMs can perform tasks such as recognizing, translating, predicting, or generating text (or other content), and can be used for text classification, question answering, document summarization, text generation, as well as plan generation, code generation, prediction problems (e.g., predicting protein structures), and so forth. Examples of LLMs include GPT-3.5, GPT-4, Bard, Cohere, PaLM, Falcon, Claude, Llama, Orca, Phi-1, Jurassic and more.

A trained neural network model (e.g., a trained machine learning trained machine-learning program 414 using a neural network 428) may be stored in a computational graph format, according to some examples. An example computational graph format is the Open Neural Network Exchange (ONNX) file format, an open, flexible standard for storing models which allows reusing models across deep learning platforms/tools, and deploying models in the cloud (e.g., via ONNX runtime).

In examples, one or more artificial intelligence agents, such as one or more machine-learned algorithms or models and/or a neural network of one or more machine-learned algorithms or models may be trained iteratively (e.g., in a plurality of stages) using a plurality of sets of input data. For example, a first set of input data may be used to train one or more of the artificial agents. Then, the first set of input data may be transformed into a second set of input data for retraining the one or more artificial intelligence agents. The continuously updated and retrained artificial intelligence agents may then be applied to subsequent novel input data to generate one or more of the outputs described herein.

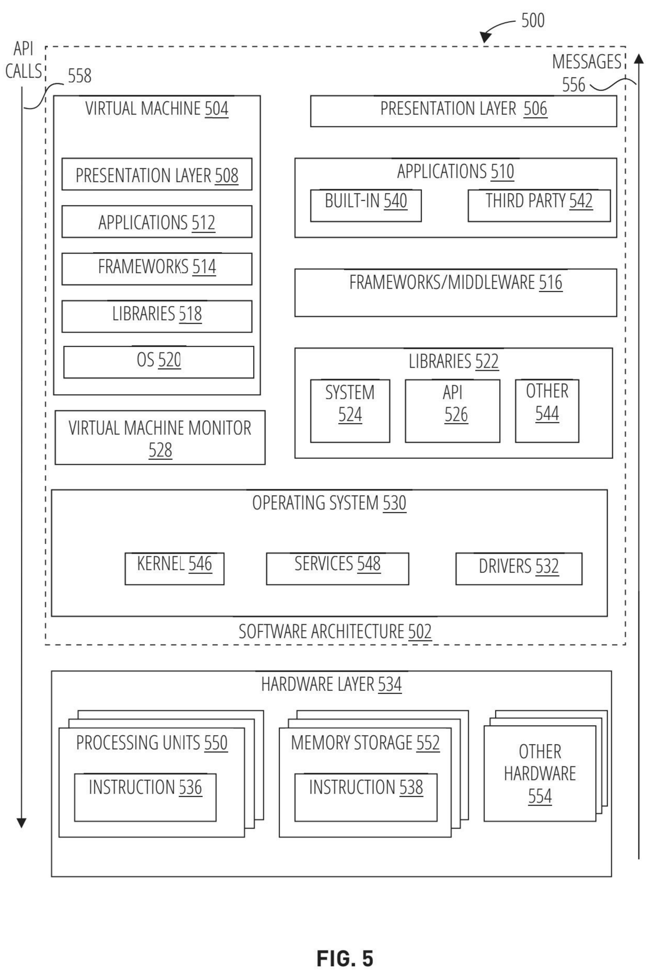

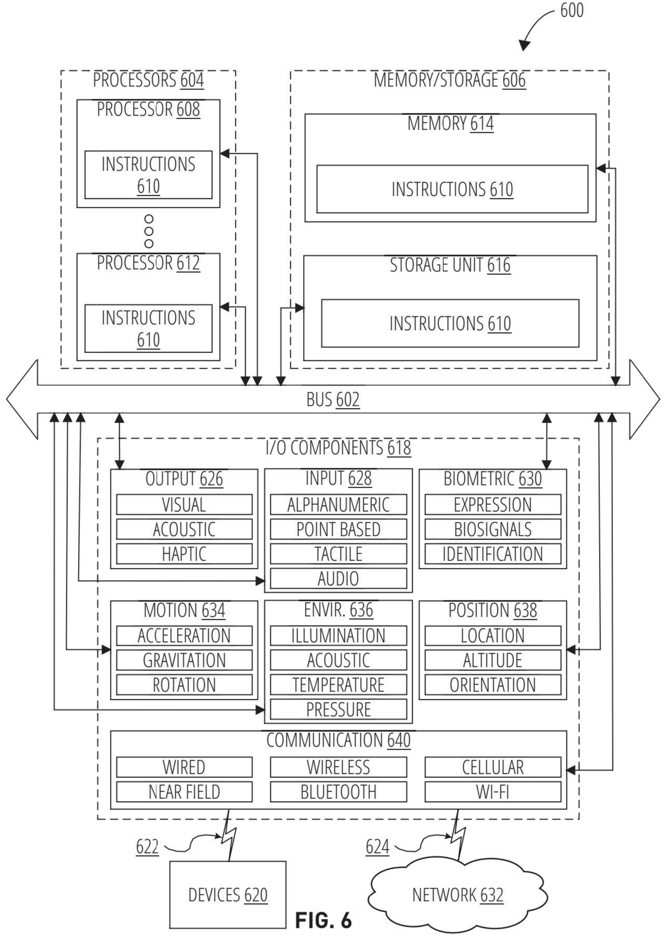

FIG. 5 is a block diagram 500 illustrating an example of a software architecture 502 that may be installed on a machine, according to some examples. FIG. 5 is merely a nonlimiting example of software architecture, and it will be appreciated that many other architectures may be implemented to facilitate the functionality described herein. The software architecture 502 may be executing on hardware such as a machine 600 of FIG. 6 that includes, among other things, processors 604, memory/storage 606, and I/O components 618. A representative hardware layer 534 is illustrated and can represent, for example, the machine of FIG. 6. The representative hardware layer 534 comprises one or more processing units 550 having associated executable instructions 536. The executable instructions 536 represent the executable instructions of the software architecture 502. The hardware layer 534 also includes memory or memory storage 552, which also have the executable instructions 538. The hardware layer 534 may also comprise other hardware 554, which represents any other hardware of the hardware layer 534 such as the other hardware illustrated as part of the machine 600.

In the example architecture of FIG. 5, the software architecture 502 may be conceptualized as a stack of layers, where each layer provides particular functionality. For example, the software architecture 502 may include layers such as an operating system 530, libraries 518, frameworks/middleware 516, applications 510, a presentation layer 508, and so forth. Operationally, the applications 510 or other components within the layers may invoke API calls through the software stack and receive a response, returned values, and so forth (illustrated as messages 556) in response to the API calls 558. The layers illustrated are representative in nature, and not all software architectures have all layers. For example, some mobile or special-purpose operating systems may not provide a frameworks/middleware 516 layer, while others may provide such a layer. Other software architectures may include additional or different layers.

The operating system 530 may manage hardware resources and provide common services. The operating system 530 may include, for example, a kernel 546, services 548, and drivers 532. The kernel 546 may act as an abstraction layer between the hardware and the other software layers. For example, the kernel 546 may be responsible for memory management, processor management (e.g., scheduling), component management, networking, security settings, and so on. The services 548 may provide other common services for the other software layers. The drivers 532 may be responsible for controlling or interfacing with the underlying hardware. For instance, the drivers 532 may include display drivers, camera drivers, Bluetooth^{®} drivers, flash memory drivers, serial communication drivers (e.g., Universal Serial Bus (USB) drivers), Wi-Fi^{®} drivers, audio drivers, power management drivers, and so forth depending on the hardware configuration.

The libraries 518 or 522 may provide a common infrastructure that may be utilized by the applications 510 and/or other components and/or layers. The libraries 518 or 522 typically provide functionality that allows other software modules to perform tasks in an easier fashion than by interfacing directly with the underlying operating system 530 functionality (e.g., kernel 546, services 548 or drivers 532). The libraries 518 or 522 may include system libraries 524 (e.g., C standard library) that may provide functions such as memory allocation functions, string manipulation functions, mathematic functions, and the like. In addition, the libraries 518 or 522 may include API libraries 526 such as media libraries (e.g., libraries to support presentation and manipulation of various media formats such as MPEG4, H.264, MP3, AAC, AMR, JPG, and PNG), graphics libraries (e.g., an OpenGL framework that may be used to render 2D and 3D graphic content on a display), database libraries (e.g., SQLite that may provide various relational database functions), web libraries (e.g., WebKit that may provide web browsing functionality), and the like. The libraries 518 or 522 may also include a wide variety of other libraries 544 to provide many other APIs to the applications 510 or applications 512 and other software components/modules.

The frameworks/middleware 516 may provide a higher-level common infrastructure that may be utilized by the applications 510 or other software components/modules. For example, the frameworks/middleware 516 may provide various graphical user interface functions, high-level resource management, high-level location services, and so forth. The frameworks/middleware 516 may provide a broad spectrum of other APIs that may be utilized by the applications 510 and/or other software components/modules, some of which may be specific to a particular operating system or platform.

The applications 510 include built-in applications 540 and/or third-party applications 542. Examples of representative built-in applications 540 may include, but are not limited to, a home application, a contacts application, a browser application, a book reader application, a location application, a media application, a messaging application, or a game application.

The third-party applications 542 may include any of the built-in applications 540 as well as a broad assortment of other applications. In a specific example, the third-party applications 542 (e.g., an application developed using the Android^{™} or iOS^{™} software development kit (SDK) by an entity other than the vendor of the particular platform) may be mobile software running on a mobile operating system such as iOS^{™}, Android^{™}, or other mobile operating systems. In this example, the third-party applications 542 may invoke the API calls 558 provided by the mobile operating system such as the operating system 530 to facilitate functionality described herein.

The applications 510 may utilize built-in operating system functions, libraries (e.g., system libraries 524, API libraries 526, and other libraries 544), or frameworks/middleware 516 to create user interfaces to interact with users of the system. Alternatively, or additionally, in some systems, interactions with a user may occur through a presentation layer, such as the presentation layer 506 or presentation layer 508. In these systems, the application/module "logic" can be separated from the aspects of the application/module that interact with the user.

Some software architectures utilize virtual machines. In the example of FIG. 5, this is illustrated by a virtual machine 504. The virtual machine 504 creates a software environment where applications/modules can execute as if they were executing on a hardware machine. The virtual machine 504 is hosted by a host operating system (e.g., the operating system 530) and typically, although not always, has a virtual machine monitor 528, which manages the operation of the virtual machine 504 as well as the interface with the host operating system (e.g., the operating system 530). A software architecture executes within the virtual machine 504, such as an operating system 520, libraries 518, frameworks/middleware 514, applications 512, or a presentation layer 508. These layers of software architecture executing within the virtual machine 504 can be the same as corresponding layers previously described or may be different.

FIG. 6 is a block diagram illustrating components of a machine 600, according to some examples, able to read instructions from a machine-readable medium (e.g., a machine-readable storage medium) and perform any one or more of the methodologies discussed herein. Specifically, FIG. 6 shows a diagrammatic representation of the machine 600 in the example form of a computer system, within which instructions 610 (e.g., software, a program, an application, an applet, an app, or other executable code) for causing the machine 600 to perform any one or more of the methodologies discussed herein may be executed. As such, the instructions 610 may be used to implement modules or components described herein. The instructions 610 transform the general, non-programmed machine 600 into a particular machine 600 programmed to carry out the described and illustrated functions in the manner described. In alternative embodiments, the machine 600 operates as a standalone device or may be coupled (e.g., networked) to other machines. In a networked deployment, the machine 600 may operate in the capacity of a server machine or a client machine in a server-client network environment, or as a peer machine in a peer-to-peer (or distributed) network environment. The machine 600 may comprise, but not be limited to, a server computer, a client computer, a personal computer (PC), a tablet computer, a laptop computer, a netbook, a personal digital assistant (PDA), an entertainment media system, a cellular telephone, a smart phone, a mobile device, a wearable device (e.g., a smart watch), other smart devices, a web appliance, a network router, a network switch, a network bridge, or any machine capable of executing the instructions 610, sequentially or otherwise, that specify actions to be taken by machine 600. Further, while only a single machine 600 is illustrated, the term "machine" shall also be taken to include a collection of machines that individually or jointly execute the instructions 610 to perform any one or more of the methodologies discussed herein.

The machine 600 may include processors 604 and 612, memory/storage 606, and I/O components 618, which may be configured to communicate with each other such as via a bus 602. The memory/storage 606 may include a memory 614, such as a main memory, or other memory storage, and a storage unit 616, both accessible to the processors 604 such as via the bus 602. The storage unit 616 and memory 614 store the instructions 610 embodying any one or more of the methodologies or functions described herein. The instructions 610 may also reside, completely or partially, within the memory 614 within the storage unit 616, within at least one of the processors 604 (e.g., within the processor's cache memory), or any suitable combination thereof, during execution thereof by the machine 600. Accordingly, the memory 614, the storage unit 616, and the memory of processors 604 are examples of machine-readable media.

The I/O components 618 may include a wide variety of components to receive input, provide output, produce output, transmit information, exchange information, capture measurements, and so on. The specific I/O components 618 that are included in a particular machine 600 will depend on the type of machine. For example, portable machines such as mobile phones will likely include a touch input device or other such input mechanisms, while a headless server machine will likely not include such a touch input device. It will be appreciated that the I/O components 618 may include many other components that are not shown in FIG. 6. The I/O components 618 are grouped according to functionality merely for simplifying the following discussion and the grouping is in no way limiting. In various examples, the I/O components 618 may include output components 626 and input components 628. The output components 626 may include visual components (e.g., a display such as a plasma display panel (PDP), a light emitting diode (LED) display, a liquid crystal display (LCD), a projector, or a cathode ray tube [CRT]), acoustic components (e.g., speakers), haptic components (e.g., a vibratory motor, resistance mechanisms), other signal generators, and so forth. The input I/O components 628 may include alphanumeric input components (e.g., a keyboard, a touch screen configured to receive alphanumeric input, a photo-optical keyboard, or other alphanumeric input components), point based input components (e.g., a mouse, a touchpad, a trackball, a joystick, a motion sensor, or other pointing instrument), tactile input components (e.g., a physical button, a touch screen that provides location and/or force of touches or touch gestures, or other tactile input components), audio input components (e.g., a microphone), and the like.

In further examples, the I/O components 618 may include biometric components 630, motion components 634, environmental components 636, or position components 638 among a wide array of other components. For example, the biometric components 630 may include components to detect expressions (e.g., hand expressions, facial expressions, vocal expressions, body gestures, or eye tracking), measure biosignals (e.g., blood pressure, heart rate, body temperature, perspiration, or brain waves), identify a person (e.g., voice identification, retinal identification, facial identification, fingerprint identification, or electroencephalogram based identification), and the like. The motion components 634 may include acceleration sensor components (e.g., accelerometer), gravitation sensor components, rotation sensor components (e.g., gyroscope), and so forth. The environment components 636 may include, for example, illumination sensor components (e.g., photometer), temperature sensor components (e.g., one or more thermometer that detect ambient temperature), humidity sensor components, pressure sensor components (e.g., barometer), acoustic sensor components (e.g., one or more microphones that detect background noise), proximity sensor components (e.g., infrared sensors that detect nearby objects), gas sensors (e.g., gas detection sensors to detection concentrations of hazardous gases for safety or to measure pollutants in the atmosphere), or other components that may provide indications, measurements, or signals corresponding to a surrounding physical environment. The position components 638 may include location sensor components (e.g., a Global Position system (GPS) receiver component), altitude sensor components (e.g., altimeters or barometers that detect air pressure from which altitude may be derived), orientation sensor components (e.g., magnetometers), and the like.

Communication may be implemented using a wide variety of technologies. The I/O components 618 may include communication components 640 operable to couple the machine 600 to a network 632 or devices 620 via coupling 624 and coupling 622 respectively. For example, the communication components 640 may include a network interface component or other suitable device to interface with the network 632. In further examples, communication components 640 may include wired communication components, wireless communication components, cellular communication components, Near Field Communication (NFC) components, Bluetooth^{®} components (e.g., Bluetooth^{®} Low Energy), Wi-Fi^{®} components, and other communication components to provide communication via other modalities. The devices 620 may be another machine or any of a wide variety of peripheral devices (e.g., a peripheral device coupled via a Universal Serial Bus [USB]).

Moreover, the communication components 640 may detect identifiers or include components operable to detect identifiers. For example, the communication components 640 may include Radio Frequency Identification (RFID) tag reader components, NFC smart tag detection components, optical reader components (e.g., an optical sensor to detect one-dimensional bar codes such as Universal Product Code (UPC) bar code, multi-dimensional bar codes such as Quick Response (QR) code, Aztec code, Data Matrix, Dataglyph, MaxiCode, PDF417, Ultra Code, UCC RSS-2D bar code, and other optical codes), or acoustic detection components (e.g., microphones to identify tagged audio signals). In addition, a variety of information may be derived via the communication components 640, such as, location via Internet Protocol (IP) geo-location, location via Wi-Fi^{®} signal triangulation, location via detecting a NFC beacon signal that may indicate a particular location, and so forth.

### EXAMPLES

Example 1 is a system comprising: at least one processor; and at least one memory storing instructions that, when executed by the at least one processor, configure the system to perform operations comprising: receive, via a query interface, a query related to a medical topic; transmit the query to one or more vector DBs; retrieve result sets from the one or more vector DBs, each result set associated with a respective vector DB of the one or more vector DBs, each result set comprising result records, each result record being associated with content and a respective content vector; normalize, based on a vectorization algorithm, one or more of the result records in the result sets to update a normalized result set; generate, based on the normalized result set, a unified result set; store the unified result set; and return the unified result set to a medical decision system that provides medical recommendations to a system user.

In Example 2, the subject matter of Example 1 includes, wherein normalizing the one or more of the result records in the result sets further comprises generating, using the vectorization algorithm, a new content vector for the content associated with each of the one or more of the result records.

In Example 3, the subject matter of Example 2 includes, wherein generating the unified result set based on the normalized result set further comprises: ranking the one or more result records in the normalized result set based on relevance to the query to generate a ranked result set; and generating the unified result set based on the ranked result set and one or more parameters.

In Example 4, the subject matter of Example 3 includes, wherein the one or more parameters further comprise a maximum result set size and a minimum set relevance score.

In Example 5, the subject matter of Examples 1-4 includes, wherein the operations further comprise: upon detecting that the vectorization algorithm matches a local vectorization algorithm of a vector DB of the one or more vector DBs, add result records of a result set retrieved from the vector DB to the normalized result set.

In Example 6, the subject matter of Examples 1-5 includes, wherein the operations further comprise: storing, using a cache, a result record associated with content and a new content vector generated using the vectorization algorithm, the cache including a cache index associated with the result record, the cache index associated with the result record corresponding to an original content vector generated based on the content and on a local vectorization algorithm for a vector DB of the one or more vector DBs; upon retrieving an additional result record from the vector DB: determining that an additional content vector associated with the additional result record corresponds to the cache index; retrieving, from the cache, the new content vector associated with the result record and the cache index; and adding the result record and the new content vector to the normalized result set.

In Example 7, the subject matter of Example 6 includes, wherein cache entries in the cache are associated with timestamps; and wherein the operations further comprise deleting cache entries based on a recency threshold and the timestamps.

Example 8 is at least one non-transitory machine-readable medium (e.g., computer-readable medium) including instructions that, when executed by processing circuitry, cause the processing circuitry to perform operations to implement of any of Examples 1-7.

Example 9 is an apparatus comprising means to implement of any of Examples 1-7.

Example 10 is a method to implement of any of Examples 1-7.

### GLOSSARY

"CARRIER SIGNAL" in this context may include any intangible medium that is capable of storing, encoding, or carrying instructions for execution by the machine, and includes digital or analog communications signals or other intangible medium to facilitate communication of such instructions. Instructions may be transmitted or received over the network using a transmission medium via a network interface device and using any one of a number of well-known transfer protocols.

"CLIENT DEVICE" in this context may include any machine that interfaces to a communications network to obtain resources from one or more server systems or other client devices. A client device may be, but is not limited to, a mobile phone, desktop computer, laptop, portable digital assistants (PDAs), smart phones, tablets, ultra books, netbooks, laptops, multi-processor systems, microprocessor-based or programmable consumer electronics, game consoles, set-top boxes, or any other communication device that a user may use to access a network.

"COMMUNICATIONS NETWORK" in this context may include one or more portions of a network that may be an ad hoc network, an intranet, an extranet, a virtual private network (VPN), a local area network (LAN), a wireless LAN (WLAN), a wide area network (WAN), a wireless WAN (WWAN), a metropolitan area network (MAN), the Internet, a portion of the Internet, a portion of the Public Switched Telephone Network (PSTN), a plain old telephone service (POTS) network, a cellular telephone network, a wireless network, a Wi-Fi^{®} network, another type of network, or a combination of two or more such networks. For example, a network or a portion of a network may include a wireless or cellular network and the coupling may be a Code Division Multiple Access (CDMA) connection, a Global System for Mobile communications (GSM) connection, or other type of cellular or wireless coupling. In this example, the coupling may implement any of a variety of types of data transfer technology, such as Single Carrier Radio Transmission Technology (1xRTT), Evolution-Data Optimized (EVDO) technology, General Packet Radio Service (GPRS) technology, Enhanced Data rates for GSM Evolution (EDGE) technology, third Generation Partnership Project (3GPP) including 3G, fourth generation wireless (4G) networks, Universal Mobile Telecommunications System (UMTS), High Speed Packet Access (HSPA), Worldwide Interoperability for Microwave Access (WiMAX), Long Term Evolution (LTE) standard, others defined by various standard setting organizations, other long range protocols, or other data transfer technology.

"MACHINE-READABLE MEDIUM" (or "computer-readable medium") in this context may include a component, device or other tangible media able to store instructions and data temporarily or permanently and may include, but is not be limited to, random-access memory (RAM), read-only memory (ROM), buffer memory, flash memory, optical media, magnetic media, cache memory, other types of storage (e.g., Erasable Programmable Read-Only Memory [EEPROM]) and/or any suitable combination thereof. The term "machine-readable medium" should be taken to include a single medium or multiple media (e.g., a centralized or distributed database, or associated caches and servers) able to store instructions. The term "machine-readable medium" shall also be taken to include any medium, or combination of multiple media, that is capable of storing instructions (e.g., code) for execution by a machine, such that the instructions, when executed by one or more processors of the machine, cause the machine to perform any one or more of the methodologies described herein. Accordingly, a "machine-readable medium" refers to a single storage apparatus or device, as well as "cloud-based" storage systems or storage networks that include multiple storage apparatus or devices. The term "machine-readable medium" excludes signals per se. The term "machine-readable medium" should be taken to refer to a non-transitory medium.

"COMPONENT" in this context may include a device, physical entity or logic having boundaries defined by function or subroutine calls, branch points, application program interfaces (APIs), or other technologies that provide for the partitioning or modularization of particular processing or control functions. Components may be combined via their interfaces with other components to carry out a machine process. A component may be a packaged functional hardware unit designed for use with other components and a part of a program that usually performs a particular function of related functions. Components may constitute either software components (e.g., code embodied on a machine-readable medium) or hardware components. A "hardware component" is a tangible unit capable of performing certain operations and may be configured or arranged in a certain physical manner. In various examples, one or more computer systems (e.g., a standalone computer system, a client computer system, or a server computer system) or one or more hardware components of a computer system (e.g., a processor or a group of processors) may be configured by software (e.g., an application or application portion) as a hardware component that operates to perform certain operations as described herein. A hardware component may also be implemented mechanically, electronically, or any suitable combination thereof. For example, a hardware component may include dedicated circuitry or logic that is permanently configured to perform certain operations. A hardware component may be a special-purpose processor, such as a Field-Programmable Gate Array (FPGA) or an Application Specific Integrated Circuit (ASIC). A hardware component may also include programmable logic or circuitry that is temporarily configured by software to perform certain operations. For example, a hardware component may include software executed by a general-purpose processor or other programmable processor. Once configured by such software, hardware components become specific machines (or specific components of a machine) uniquely tailored to perform the configured functions and are no longer general-purpose processors. It will be appreciated that the decision to implement a hardware component mechanically, in dedicated and permanently configured circuitry, or in temporarily configured circuitry (e.g., configured by software) may be driven by cost and time considerations. Accordingly, the phrase "hardware component"(or "hardware-implemented component") should be understood to encompass a tangible entity, be that an entity that is physically constructed, permanently configured (e.g., hardwired), or temporarily configured (e.g., programmed) to operate in a certain manner or to perform certain operations described herein. Considering embodiments in which hardware components are temporarily configured (e.g., programmed), each of the hardware components need not be configured or instantiated at any one instance in time. For example, where a hardware component comprises a general-purpose processor configured by software to become a special-purpose processor, the general-purpose processor may be configured as respectively different special-purpose processors (e.g., comprising different hardware components) at different times. Software accordingly configures a particular processor or processors, for example, to constitute a particular hardware component at one instance of time and to constitute a different hardware component at a different instance of time. Hardware components can provide information to, and receive information from, other hardware components. Accordingly, the described hardware components may be regarded as being communicatively coupled. Where multiple hardware components exist contemporaneously, communications may be achieved through signal transmission (e.g., over appropriate circuits and buses) between or among two or more of the hardware components. In embodiments in which multiple hardware components are configured or instantiated at different times, communications between such hardware components may be achieved, for example, through the storage and retrieval of information in memory structures to which the multiple hardware components have access. For example, one hardware component may perform an operation and store the output of that operation in a memory device to which it is communicatively coupled. A further hardware component may then, at a later time, access the memory device to retrieve and process the stored output. Hardware components may also initiate communications with input or output devices, and can operate on a resource (e.g., a collection of information). The various operations of example methods described herein may be performed, at least partially, by one or more processors that are temporarily configured (e.g., by software) or permanently configured to perform the relevant operations. Whether temporarily or permanently configured, such processors may constitute processor-implemented components that operate to perform one or more operations or functions described herein. As used herein, "processor-implemented component" refers to a hardware component implemented using one or more processors. Similarly, the methods described herein may be at least partially processor-implemented, with a particular processor or processors being an example of hardware. For example, at least some of the operations of a method may be performed by one or more processors or processor-implemented components. Moreover, the one or more processors may also operate to support performance of the relevant operations in a "cloud computing" environment or as a "software as a service" (SaaS). For example, at least some of the operations may be performed by a group of computers (as examples of machines including processors), with these operations being accessible via a network (e.g., the Internet) and via one or more appropriate interfaces (e.g., an Application Program Interface [API]). The performance of certain of the operations may be distributed among the processors, not only residing within a single machine, but deployed across a number of machines. In some examples, the processors or processor-implemented components may be located in a single geographic location (e.g., within a home environment, an office environment, or a server farm). In other examples, the processors or processor-implemented components may be distributed across a number of geographic locations.

"PROCESSOR" in this context may include any circuit or virtual circuit (a physical circuit emulated by logic executing on an actual processor) that manipulates data values according to control signals (e.g., "commands", "op codes", "machine code", etc. ) and which produces corresponding output signals that are applied to operate a machine. A processor may, for example, be a Central Processing Unit (CPU), a Reduced Instruction Set Computing (RISC) processor, a Complex Instruction Set Computing (CISC) processor, a Graphics Processing Unit (GPU), a Digital Signal Processor (DSP), an Application Specific Integrated Circuit (ASIC), a Radio-Frequency Integrated Circuit (RFIC) or any combination thereof. A processor may further be a multi-core processor having two or more independent processors (sometimes referred to as "cores") that may execute instructions contemporaneously.

"TIMESTAMP" in this context may include a sequence of characters or encoded information identifying when a certain event occurred, for example giving date and time of day, sometimes accurate to a small fraction of a second.

"TIME DELAYED NEURAL NETWORK (TDNN)" in this context may include an artificial neural network architecture whose primary purpose is to work on sequential data. An example would be converting continuous audio into a stream of classified phoneme labels for speech recognition.

"BI-DIRECTIONAL LONG-SHORT TERM MEMORY (BLSTM)" in this context may include a recurrent neural network (RNN) architecture that remembers values over arbitrary intervals. Stored values are not modified as learning proceeds. RNNs allow forward and backward connections between neurons. BLSTM are well-suited for the classification, processing, and prediction of time series, given time lags of unknown size and duration between events.

### NOTES

Although example methods disclosed herein depict particular sequences of operations, the sequences may be altered without departing from the scope of the present disclosure. For example, some of the operations depicted may be performed in parallel or in a different sequence that does not materially affect the function of a respective method. In other examples, different components of an example device or system that implements the method may perform functions at substantially the same time or in a specific sequence.

## Claims

1. A system comprising:
at least one processor; and
at least one memory storing instructions that, when executed by the at least one processor, configure the system to perform operations comprising:
receive, via a query interface, a query related to a medical topic;
transmit the query to one or more vector DBs;
retrieve result sets from the one or more vector DBs, each result set associated with a respective vector DB of the one or more vector DBs, each result set comprising result records, each result record being associated with content and a respective content vector;
normalize, based on a vectorization algorithm, one or more of the result records in the result sets to update a normalized result set;
generate, based on the normalized result set, a unified result set;
store the unified result set; and
return the unified result set to a medical decision system that provides medical recommendations to a system user.

2. The system of claim 1, wherein normalizing the one or more of the result records in the result sets further comprises generating, using the vectorization algorithm, a new content vector for the content associated with each of the one or more of the result records.

3. The system of claim 2, wherein generating the unified result set based on the normalized result set further comprises:
ranking the one or more result records in the normalized result set based on relevance to the query to generate a ranked result set; and
generating the unified result set based on the ranked result set and one or more parameters.

4. The system of claim 3, wherein the one or more parameters further comprise a maximum result set size and a minimum set relevance score.

5. The system of claim 1, wherein the operations further comprise:
upon detecting that the vectorization algorithm matches a local vectorization algorithm of a vector DB of the one or more vector DBs, add result records of a result set retrieved from the vector DB to the normalized result set.

6. The system of claim 1, wherein the operations further comprise:
storing, using a cache, a result record associated with content and a new content vector generated using the vectorization algorithm, the cache including a cache index associated with the result record, the cache index associated with the result record corresponding to an original content vector generated based on the content and on a local vectorization algorithm for a vector DB of the one or more vector DBs;
upon retrieving an additional result record from the vector DB:
determining that an additional content vector associated with the additional result record corresponds to the cache index;
retrieving, from the cache, the new content vector associated with the result record and the cache index; and
adding the result record and the new content vector to the normalized result set.

7. The system of claim 6, wherein cache entries in the cache are associated with timestamps; and wherein the operations further comprise deleting cache entries based on a recency threshold and the timestamps.

8. A method comprising:
receiving via a query interface, a query related to a medical topic;
transmitting the query to one or more vector DBs;
retrieving result sets from the one or more vector DBs, each result set associated with a respective vector DB of the one or more vector DBs, each result set comprising result records, each result record being associated with content and a respective content vector;
normalizing, based on a vectorization algorithm, one or more of the result records in the result sets to update a normalized result set;
generating, based on the normalized result set, a unified result set;
storing the unified result set; and
returning the unified result set to a medical decision system that provides medical recommendations to a system user.

9. The method of claim 8, wherein normalizing the one or more of the result records in the result sets further comprises generating, using the vectorization algorithm, a new content vector for the content associated with each of the one or more of the result records.

10. The method of claim 9, wherein generating the unified result set based on the normalized result set further comprises:
ranking the one or more result records in the normalized result set based on relevance to the query to generate a ranked result set; and
generating the unified result set based on the ranked result set and one or more parameters.

11. The method of claim 10, wherein the one or more parameters further comprise a maximum result set size and a minimum set relevance score.

12. The method of claim 8, further comprising: upon detecting that the vectorization algorithm matches a local vectorization algorithm of a vector DB of the one or more vector DBs, adding result records of a result set retrieved from the vector DB to the normalized result set.

13. The method of claim 8, further comprising:
storing, using a cache, a result record associated with content and a new content vector generated using the vectorization algorithm, the cache including a cache index associated with the result record, the cache index corresponding to an original content vector generated based on the content and on a local vectorization algorithm for a vector DB of the one or more vector DBs; and
upon retrieving an additional result record from the vector DB:
determining that an additional content vector associated with the additional result record corresponds to the cache index;
retrieving, from the cache, the new content vector associated with the result record and the cache index; and
adding the result record and the new content vector to the normalized result set.

14. The method of claim 13, wherein cache entries in the cache are associated with timestamps, and wherein the method further comprises deleting cache entries based on a recency threshold and the timestamps.

15. A computer-readable storage medium, the computer-readable storage medium including instructions that when executed by a computer, cause the computer to perform the method of any one of claims 8 to 14.
